# EUROPÄISCHE PATENTSCHRIFT

(11) **EP 1 015 108 B1**
(45) Veröffentlichungstag und Bekanntmachung des Hinweises auf die Patenterteilung: **13.06.2001**
(21) Anmeldenummer: 98946269.2
(22) Anmeldetag: 01.08.1998
(51) Int. Cl.: B01J 37/02, B01J 37/16, C07C 67/055

(54) **VERFAHREN ZUR HERSTELLUNG VON PORÖS GETRÄGERTEN METALL-NANOPARTIKELHALTIGEN KATALYSATOREN, INSBESONDERE FÜR DIE GASPHASENOXIDATION VON ETHYLEN UND ESSIGSÄURE ZU VINYLACETAT**
METHOD FOR PRODUCING CATALYSTS CONTAINING METAL NANOPARTICLES ON A POROUS SUPPORT, ESPECIALLY FOR GAS PHASE OXIDATION OF ETHYLENE AND ACETIC ACID TO FORM VINYL ACETATE
PROCEDE DE PRODUCTION DE CATALYSEURS CONTENANT DES NANOPARTICULES METALLIQUES A SUPPORT POREUX, EN PARTICULIER POUR L'OXYDATION EN PHASE GAZEUSE D'ETHYLENE ET D'ACIDE ACETIQUE EN ACETATE DE VINYLE

(30) Priorität: 13.08.1997 DE 19734974
(43) Veröffentlichungstag der Anmeldung: 05.07.2000
(73) Patentinhaber: Celanese Chemicals Europe GmbH, 60439 Frankfurt am Main (DE)
(72) Erfinder: HAGEMEYER, Alfred, D-65929 Frankfurt am Main (DE); DINGERDISSEN, Uwe, D-64342 Seeheim-Jugenheim (DE); KÜHLEIN, Klaus, D-65779 Kelkheim (DE); MANZ, Andreas, D-76547 Sinzheim (DE); FISCHER, Roland, D-69151 Neckargemünd (DE)
(86) Internationale Anmeldenummer: EP9804819
(87) Internationale Veröffentlichungsnummer: WO9908791

(56) Entgegenhaltungen:
- EP-A- 0 012 153
- EP-A- 0 576 828
- EP-A- 0 634 208
- WO-A-94/08714
- WO-A-97/36678
- GB-A- 2 006 261

## Beschreibung

Die Erfindung betrifft ein Verfahren zur Herstellung eines auf porösen Trägerteilchen ein oder mehrere Metalle aus der Gruppe von Metallen, welche die Nebengruppen Ib und VIIIb des periodischen Systems der Elemente umfaßt, aufweisenden Katalysators. Dabei liegen die Metalle im fertigen Katalysator als Nanopartikel vor. Insbesondere bezieht sich die Erfindung auf die Herstellung von Schalenkatalysatoren auf porösen, vorzugsweise nanoporösen, Trägern nach dieser Methode.

Katalysatoren, vorzugsweise Schalenkatalysatoren, können für eine Vielzahl von heterogen katalysierten Reaktionen wie Hydrierungen und Oxidationen eingesetzt werden. Unter anderem eignen sich Pd/Au-Schalenkatalysatoren vorzüglich für die Katalyse der Gasphasenoxidation von Ethylen und Essigsäure zu Vinylacetat. Dabei werden die katalytisch aktiven Metalle schalenförmig auf oder in der äußersten Schicht des Trägers abgeschieden. Ihre Herstellung erfolgt zum Teil durch Penetration des Trägers mit Metallsalzen bis in einen oberflächennahen Bereich und anschließende Fällung durch Alkalien zu wasserunlöslichen Pd/Au-Verbindungen.

GB-A-1 283 737 offenbart die Herstellung eines Edelmetall-Schalenkatalysators durch Vortränkung des Trägers mit einer alkalischen Lösung und Sättigung mit 25-90% Wasser oder Alkohol, Die anschließende Imprägnierung mit Pd-Salzen und die Reduktion der abgeschiedenen Salze zum Metall ergibt Schalenkatalysatoren, wobei die Eindringtiefe der Edelmetalle bis zu 50% des Pelletradius betragen soll.

Gemäß den US-PSen 3,775,342 und 3,822,308 erzeugt man Schalenkatalysatoren durch Tränkung des Trägers mit einer Lösung aus Pd/Au-Salzen und mit einer wässrigen Base, vorzugsweise NaOH, wobei unlösliche Pd- und Au-Hydroxide in einer schalenförmigen Oberflächenzone auf den Pellets ausfallen. Die auf diese Weise in der Schale fixierten Hydroxide werden dann zu den Metallen reduziert.

Die GB-A-1 521 652 erhält nach einer vergleichbaren Vorgehensweise (Vorimprägnierung mit Pd-,Au-Salzen, Trocknung, Basenfällung, Reduktion) Schalenkatalysatoren vom eggwhite-Typ, d.h. nur ein innerer Ring des kugelförmigen SiO₂-Trägers enthält die Edelmetalle, während der innere Kern und eine dünne äußere Schale nahezu edelmetallfrei bleiben.

Die US-PS 4,048,096 lehrt die Fällung wasserunlöslicher Pd- und Au-Verbindungen auf dem mit Pd/Au-Salzen vorimprägnierten Träger mit Na-Silikaten anstelle von NaOH. Die Schalendicke beträgt dabei weniger als 0,5 mm.

US-PS 5,567,839 fällt wasserunlösliche Pd- und Au-Verbindungen auf dem mit Pd/Au-Salzen vorimprägnierten Träger mit Ba-Hydroxid anstelle von NaOH. Die Schalendicke liegt bei 1 mm. Der Katalysator kann weiterhin mit Ba-Acetat dotiert sein.

Aus EP-A-0 519 435 ist die Herstellung eines Pd/Au/K- oder Pd/Cd/K-Schalenkatalysators bekannt, wobei ein spezielles Trägermaterial vor der Imprägnierung mit einer Säure gewaschen und nach der Imprägnierung mit einer Base behandelt wird.

In der US-PS 5,314,858 wird die zweifache Fixierung der Edelmetalle in einer äußeren Schale durch zwei separate Fällschritte mit NaOH diskutiert.

WO-A-94/08714 erzielt durch rotatorische Bewegung des mit Pd-Au-Salzen imprägnierten Trägers während des Fixierungsschrittes, d.h. eingetaucht in die alkalische Fixierlösung (NaOH), besonders gleichmäßige Schalen.

EP-A-0 723 810 stellt durch Vorbehandlung (Tränkung) des Trägers mit Metallsalzlösungen einen bevorzugt mit Al, Zr, Ti dotierten Träger her, der anschließend für die oben beschriebene Basenfällung zur Ausbildung eines Pd/Au/K-Schalenkatalysators eingesetzt wird.

US-PS 5,347,046 beschreibt die Promotierung von Pd/Au-Systemen mit Cu, Ni, Co, Fe, Mn, Pb, Ag auf durch Alkali-Hydroxid und Alkali-Silikat vorbehandelten SiO₂-Trägern.

Eine andere Methode zur Erzeugung von Schalenkatalysatoren besteht in der Vorbekeimung mit Metallen und anschließend der Abscheidung der vorgesehenen Menge Edelmetalle.

Die offengelegte japanische Patentanmeldung 48-10135/1973 beschreibt die Herstellung eines Pd/Au-Schalenkatalysators. Dabei wird in einem Vorbehandlungsschritt eine kleine Menge an reduziertem Metall (Gold) auf dem porösen Träger abgeschieden. Durch nachfolgende Imprägnierung wird Pd in einer Oberflächenzone mit einer Dicke von ca. 15% des Partikelradius abgeschieden.

US-PS 4,087,622 lehrt die Herstellung von Schalenkatalysatoren durch Vorbekeimung mit (reduzierten) Pd/Au-Metallkeimen in geringer Konzentration, indem der poröse SiO₂- oder Al₂O₃-Träger mit einer Pd/Au-Salzlösung imprägniert wird, getrocknet wird und dann das Pd/Au-Salz zum Metall reduziert wird. Dieser Prenukleationsschritt wird gefolgt von der Abscheidung der katalytisch notwendigen Edelmetallmenge, also der Hauptmenge, die sich dann in einer oberflächennahen Schale anreichert.

Durch Anwendung verschiedener Varianten von "Unterschußtechniken" laßen sich ebenfalls Schalenkatalysatoren erhalten. Hierzu gehören unter anderem:
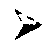 Unterschuß von Fällungsmitteln, wie NaOH, in Kombination mit Mehrfachfällung;
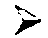 Unterschuß an Tränklösung (weniger als Porenvolumen des Trägers);
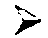 Begrenzung der Einwirkzeit beim Aufziehen der Edelmetalle;
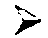 Unterschuß der Edelmetallkonzentration (pro Tränkschritt) kombiniert mit Mehrfachtränkung; und
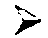 Kombinationen der vorgenannten Varianten.

In EP-A-0 565 952 wird beschrieben, daß man schalenförmig aufgebaute Pd/K/Au-, Pd/K/Ba- bzw. Pd/K/Cd-Katalysatoren erhält, wenn man eine Lösung entsprechender Metallsalze mittels Ultraschall zerstäubt und dann in einer derart beschränkten Menge und innerhalb einer derart beschränkten Zeit auf die Trägerteilchen aufbringt und mit deren Trocknung beginnt, daß die katalytisch wirksamen Metallsalze nicht bis zum Kern in die Trägerteilchen eindringen können, sondern nur in einen mehr oder weniger großen äußeren Teil, die sogenannte Schale.

Nach EP-A-0 634 214 erhält man Schalenkatalysatoren, indem eine viskose Lösung entsprechender Metallsalze in Form von Tropfen oder Flüssigkeitsstrahlen auf die Trägerteilchen aufgespritzt wird, wobei das Lösungsvolumen bei jedem Bespritzen 5-80% des Porenvolumens der Trägerteilchen beträgt und unmittelbar nach dem Aufspritzen die Trocknung eingeleitet wird.

EP-A-0 634 209 erhält Schalenkatalysatoren, indem die Trägerteilchen mit einer viskosen Lösung entsprechender Metallsalze getränkt werden, wobei das Lösungsvolumen bei jedem Tränkschritt 5-80% des Porenvolumens der Trägerteilchen beträgt und unmittelbar nach jedem Tränkschritt die Trocknung eingeleitet wird.

Gemäß EP-A-0 634 208 erhält man Schalenkatalysatoren, indem man die Trägerteilchen mit einer viskosen Lösung von Salzen der entsprechenden Elemente tränkt und dann trocknet, wobei das Lösungsvolumen beim Tränken mehr als 80% des Porenvolumens der Trägerteilchen beträgt und die Dauer der Tränkung sowie die Zeit bis zum Beginn der Trocknung so kurz gewählt werden, daß nach Ende der Trocknung eine Schale von 5 - 80% des Porenvolumens der Trägerteilchen die genannten Metallsalze enthält.

US-PS 5,576,457 behandelt Pd/Cd/K-Schalenkatalysatoren, die mit Zr und/oder Re dotiert sind, wobei die Schale nach EP 0634208, EP 0634209 oder EP 0634214 erzeugt werden kann.

US-PS 5,591,688 beschreibt Wirbelschicht-VAM-Katalysatoren (VAM = Vinylacetat) aus Pd-Ba, Au, La, Nb, Ce, Zn, Pb, Ca, Sr, Sb auf "silica", "alumina" oder "zirconia", wobei Halogenid-freie Precursoren zum Einsatz kommen.

US-PS 5,536,693 beschreibt Wirbelschicht-VAM-Katalysatoren aus Pd-Au, Cd, Bi, Cu, Mn, Fe, Co, Ce, U, die durch Mahlen eines mit Pd-M vorimprägnierten Festbett-Katalysatorprecursors und Verkitten mit einem Binder aus "silica", "alumina", "zirconia", "titania" hergestellt werden.

Aus GB-A-2 006 261 sind Rh-haltige Katalysatoren für die Fischer-Tropsch-Synthese bekannt.

Die Herstellung und Stabilisierung von Edelmetall-Nanoteilchen in Lösung gehört bereits zum Stand der Technik. Andere geläufige Bezeichnungen für solche Lösungen sind Sole oder Kolloide. Eine zusammenfassende Übersicht findet sich in G. Schmidt, Cluster and Colloids, From Theory to Applications, VCH Weinheim 1994.

Stabilie Sole werden durch Reduktion von Metallsalzlösungen mit einem Reduktionsmittel in Gegenwart eines Stabilisators hergestellt, der die Nanoteilchen umhüllt und die weitere Agglomeration der Nanoteilchen verhindert.

Bei geeigneter Wahl des Reduktionsmittels und Stabilisators können monomodale Sole mit enger Partikelgrößenverteilung hergestellt werden. Die resultierenden Teilchengrößen bewegen sich im Bereich von < 200 nm.

Ebenfalls bekannt ist die Soltränktechnik zum Aufbringen der Sole aus wässrigen Lösungen auf Träger.

So beschreibt beispielsweise DE-A 195 00 366 die Herstellung von Pd-Schalenkatalysatoren für Hydrierungen, indem man das Pd als hochverdünntes Sol durch Tränkung oder durch Aufsprühen auf einen Träger aufbringt, wobei eine Schalendicke von weniger als 5 µm resultiert.

Diese geringe Schalendicke ist für viele Hydrierreaktionen unkritisch, kann aber für andere Reaktionen, wie z. B. die VAM-Synthese, problematisch sein, da der sehr geringe Edelmetallgehalt zu Aktivitätseinbußen führt. Wünschenswert wären hier Schalen im Bereich von 5 - 1000 µm, die eine ausreichend hohe Edelmetallmenge aufnehmen können. Der Pd-Gehalt liegt bei VAM-Katalysatoren im Bereich von 1 Gew.-% und damit sehr hoch im Vergleich zu Hydrierkontakten (0.05 - 0.5 Gew.-%).

Michel und Schwartz beschreiben in "Catalyst Preparation Science IV" (Eds.: Delmon, Grange, Jacobs, Poncelet), Elsevier Science Publishers, New York, 1987, pp.669-687, die Herstellung von bimetallischen monodispersen Pd-Au Nanoteilchen mit 3 verschiedenen Mikrostrukturen (Legierung, Au-Schale auf Pd-Kern und vice versa) und deren Trägerung auf Kohlenstoff durch Adsorption aus der kolloidalen Lösung.

Schmid, West, Malm, Bovin, Grenthe (Chem. Eur. J., 1996, 2, No.9, 1099) stellen Pd/Au-Katalysatoren für die Hydrierung von Hexin duch Dipcoating eines TiO₂-Trägers in kolloidalen Pd/Au-Lösungen her.

DE-A 44 43 705 beschreibt die Herstellung von tensidstabilisierten Mono- und Bimetallkolloiden als isolierbare und in hoher Konzentration wasserlösliche Precursoren, die anschließend zur adsorptiven Belegung von Katalysatorträgern aus wäßriger Lösung eingesetzt werden.

DE-A 44 43 701 beschreibt Schalenkatalysatoren, die durch Belegen der Träger mit den katalytisch aktiven Metallen in wäßrigen Lösungen von mono- oder bimetallischen Kolloiden dieser Metalle erhalten werden, wobei die Kolloide durch stark hydrophile Tenside stabilisert sind.

Die Soltränktechnik basiert also auf einer zwei-Schritte-Prozedur, nämlich der Herstellung der Sole durch einen Reduktionsschritt und, ggf. nach weiteren Isolierungs- und Reinigungsschritten, deren nachfolgende Trägerfixierung. Dieses mehrere Schritte beinhaltende Verfahren ist an sich relativ aufwendig.

Mithin war es eine Aufgabe der vorliegenden Erfindung, ein einfaches Verfahren zur Herstellung eines auf porösen Trägerteilchen ein oder mehrere Metalle aus der Gruppe von Metallen, welche die Nebengruppen Ib und VIIIb des periodischen Systems der Elemente, mit Ausnahme von Ru, umfaßt, aufweisenden Katalysators anzugeben.

Eine weitere Aufgabe der Erfindung bestand darin, ein ohne großen Aufwand durchführbares Herstellverfahren für Sol-beschichtete Trägerkatalysatoren bereitzustellen.

Noch eine Aufgabe der Erfindung war es, ein verbessertes Verfahren für die Herstellung von Schalenkatalysatoren auf porösen, vorzugsweise nanoporösen, Trägern anzugeben, bei dem die resultierenden Schalendicken ausreichend auch für die Vinylacetat-Herstellung (VAM-Synthese) sein sollen.

Weiters lag der Erfindung die Aufgabe zugrunde, einen aktiven und selektiven VAM-Schalenkatalysator auf Basis Pd/Au mit wenigen Arbeitsschritten, schnell und kostengünstig herzustellen und dabei eine leichte Steuerbarkeit der Schalendicke zu ermöglichen.

Gelöst werden diese sowie weitere nicht näher aufgezählte, jedoch aus der einleitenden Erörterung des Standes der Technik ableitbare oder erschließbare Aufgaben durch ein Verfahren der eingangs erwähnten Art mit den Merkmalen des kennzeichnenden Teils des Anspruches 1. Vorteilhafte Modifikationen des Verfahrens der Erfindung werden in den von Anspruch 1 abhängigen Unteransprüchen unter Schutz gestellt.

Dadurch, daß man bei einem Verfahren zur Herstellung eines auf porösen Trägerteilchen ein oder mehrere Metalle aus der Gruppe von Metallen, welche die Nebengruppen Ib und VIIIb des periodischen Sytems der Elemente, mit Ausnahme von Ru, umfaßt, aufweisenden Katalysators,
in einem ersten Schritt einen porösen Träger mit einem oder mehreren Precursor(en) aus der Gruppe der Verbindungen, welche die Verbindungen der Metalle aus den Nebengruppen Ib und VIIIb des Periodensystems, mit Ausnahme von Ru, umfaßt, beaufschlagt; und
in einem zweiten Schritt den mit wenigstens einem Precursor beaufschlagten porösen Träger mit wenigstens einem Reduktionsmittel unter Erhalt von in den Poren des Trägers in situ hergestellten metallenen Nanopartikeln behandelt;
gelingt es besonders vorteilhaft ein Verfahren bereitzustellen, welches die bekannten Verfahren sowohl hinsichtlich der Einfachheit der Durchführung sowie der universellen Anwendbarkeit als auch der Qualität der resultierenden Verfahrensprodukte in nicht ohne weiteres vorhersehbarer Weise verbessert.

Bei Durchführung der Erfindung ergeben sich eine ganze Reihe von Vorteilen verglichen mit den bekannten Methoden:
⇒ So wird anstelle der aufwendigen Soltränktechnik (mit den Schritten: Solherstellung, Beladung des Trägers, Fixierung) ein sehr einfaches weniger Schritte umfassendes Verfahren angewendet, in welchem die Sole in-situ in den Poren des Trägers durch Reduktion hergestellt werden. Hierbei gelingt die Herstellung der Sole und deren Trägerfixierung simultan in einer "Eintopfreaktion" mit weniger Schritten oder Stufen als bei den aus dem Stand der Technik bekannten Verfahren.
⇒ Insbesondere der herkömmlicherweise noch erforderliche nachgeschaltete Reduktionsschritt entfällt beim erfindungsgemäßen Verfahren, da die Ausbildung der Schalenstruktur und die Reduktion zu den Metallen gleichzeitig in einem Schritt erfolgen.
⇒ Auf die erfindungsgemäße Art können auf einfache Weise Schalenkatalysatoren erhalten werden, deren Schalendicke den Erfordernissen leichter angepaßt werden kann als bei bekannten Techniken.
⇒ Insbesondere sind auch - falls gewünscht - größere Schalendicken möglich als bei der herkömmlichen Soltränktechnik, bei der die Diffusion der Sole von außen in die Trägerporen auch durch den mechanischem Siebeffekt behindert ist.
⇒ Eine schalenförmige Beladung mit Metallsalzen bei der Vorimprägnierung nach bekannten Techniken sowie ein rascher Wasserentzug beim Trocknen z.B. im Vakuum fördern zusätzlich zur erfindungsgemäßen Reduktionsmethode die Ausbildung von Schalen bzw. erlauben eine weitere Verringerung der Schalendicke, falls dies erwünscht ist.
⇒ Weiters sind nach dem erfindungsgemäßen Verfahren höhere Edelmetallbeladungen auf dem Träger möglich, es werden Arbeitsschritte eingespart und das energieintensive Handling mit hochverdünnten Lösungen wird vermieden.
⇒ Die Erfindung ermöglicht Katalysatorpartikel von wesentlich besserer Einheitlichkeit, eine im wesentlichen monomodale und schmalbandigere Partikelgrößenverteilung und kleinere Partikelgrößen verglichen mit konventionellen Präparationstechniken
⇒ Überaus vorteilhaft kann - im Falle einer wohldefinierten Porenstruktur des Trägers - über die Porengröße des Trägers die Kolloidgröße exakt eingestellt werden, so daß monomodale Verteilungen von Kolloiden einfacher hergestellt werden können.
⇒ Bei den bekannten Techniken führen Verunreinigungen in Solen zu größeren Teilchengrößen sowie zur Agglomeration von Teilchen. Im Gegensatz dazu können die zur Präparation von Solen benötigten peinlichst sauberen Apparaturen und Lösungsmittel (bidest. Wasser) bei der Verfahrensweise der Erfindung, d.h. bei der in-situ Herstellung, vollkommen entfallen.
⇒ Im Fall der VAM-Katalysatoren hat die Erfindung gegenüber dem technisch angewandten Verfahren der Fällung von Edelmetallhydroxiden mit NaOH gefolgt von einem Reduktionsschritt den Vorteil einer enormen Zeitersparnis (und damit Kostenersparnis) bei der Herstellung: Denn erfindungsgemäß kann die Schale in wenigen Minuten erzeugt werden, während sich die NaOH-Fällung über mehr als 20 h erstreckt.
⇒ Nach dem Verfahren der Erfindung erhältliche Schalenkatalysatoren zeichnen sich aufgrund der möglichen geringen Partikelgröße, Einheitlichkeit der Partikelgrößenverteilung und möglichen großen Schalendicke durch hohe Aktivitäten und Selektivitäten und durch hohe Langzeitbeständigkeit aus.

Die Poren in einer Oberflächenzone des Trägersystems werden in der vorliegenden Erfindung als "Mikroreaktoren" zur in-situ Synthese von, gegebenenfalls stabilisierten, Kolloiden benutzt, die nach der endgültigen Trocknung als feindisperse Nanoteilchen auf dem Träger fixiert sind.

Als Trägermaterialien sind daher im Verfahren der Erfindung alle porösen Materialien einsetzbar, die über eine geeignete Porosität verfügen, also mikroporös, nanoporös oder mesoporös sind, und in bezug auf den beabsichtigten Einsatzzweck sowie das Herstellungsverfahren im wesentlichen inert sind. Die Form der Trägermaterialien ist beliebig und an den Einsatzzweck anpaßbar.

In zweckmäßiger Verfahrensabwandlung kennzeichnet sich das Verfahren der Erfindung dadurch, daß man einen inerten, porösen, vorzugsweise nanoporösen, Träger aus Siliciumdioxid, Aluminiumoxid, Titandioxid, Zirkondioxid, Oxidgemischen der genannten Verbindungen, Mischoxiden der genannten Verbindungen und/oder Aluminiumsilikaten in Form von Pulvern, Folien, Bändern, Membranen, Strängen, Platten, Tabletten, Wagenrädern, Monolithen, Kugeln, Splittern, Ringen, Voll-Extrudaten, Hohl-Extrudaten, Sternen oder anderen Formkörpern einsetzt.

Besonders zweckmäßig werden als Träger SiO₂, Al₂O₃, Mischoxide aus SiO₂ und Al₂O₃ oder Gemische dieser Oxide in Form von Kugeln, Tabletten, Ringen, Sternen oder anderen Formkörpern benutzt.

Der Durchmesser bzw. die Länge und Dicke der Trägerteilchen liegt im allgemeinen bei 3 bis 9 mm. Die Oberfläche der Träger liegt, gemessen mit der BET-Methode, im allgemeinen bei 10 - 500 m²/g, bevorzugt bei 20 - 250 m²/g. Das Porenvolumen liegt im allgemeinen bei 0.3 bis 1.2 ml/g.

Von besonderem Interesse sind poröse, vorzugsweise nanoporöse, Aluminiumoxid-Träger, beispielsweise in Form von Membranen, Tabletten, Kugeln oder Pulvern.

Die hierin als bevorzugt gekennzeichneten nanoporösen Trägermaterialien sind ebenso wie die mikro- oder mesoporösen Träger an sich bekannt.

So sind beispielsweise nanoporöse Aluminiumoxid-Träger-Membranen auch im Handel erhältlich: Sie weisen in regelmäßiger Anordnung Nanoporen mit einer Porenweite im Bereich von etwa 1 bis 500 nm und einer Tiefe von bis zu 500 µm auf. Die Porendichte liegt üblicherweise im Bereich von 109 bis 1012 Poren/cm².

Übersichten über die Struktur, Herstellung und Eigenschaften porenförmiger anodischer Oxidfilme vermitteln J.W. Diggle et al., Chem. Rev. 69, 365-405 (1969) und J.P. Gullivan et al., Proceeding of the Royal Society of London, 317 (1970), 51 ff.; weitere Hinweise sind zu entnehmen bei C.A. Foss et al. J. Phys. Chem. (1994), 98, 2963-2971 und C.K. Preston et al., J. Phys. Chem. (1993), 97, 8495-8503.

Die nanoporösen Strukturen lassen sich grundsätzlich und bevorzugt durch anodische Oxidation von Metalloberflächen, vorzugsweise Aluminiumoberflächen, in einer eine di- oder triprotische Säure enthaltenden wäßrigen Lösung erzeugen.

Als Säure hierfür kommen insbesondere Schwefelsäure, Oxalsäure, Phosphorsäure und Chromsäure infrage. Die anodische Oxidation von Aluminium zur Herstellung der erfindungsgemäß einzusetzenden Membranen wird üblicherweise bei niedriger Temperatur, etwa 0 bis 5 °C, und vorzugsweise unter Einsatz von Schwefelsäure oder Oxalsäure als Elektrolyt durchgeführt, weil auf diese Weise dicke und harte poröse Filme erhältlich sind. Bei der Herstellung der Filme bildet beispielsweise ein Blech aus hochreinem Aluminium die Anode in einer elektrochemischen Zelle. Die Anodisierung verläuft unter genauer Potential- und Stromkontrolle ab. Der Porendurchmesser ist abhängig vom Elektrolyt, der Temperatur und der Anodisierungsspannung, wobei mit zunehmender Spannung der Durchmesser zunimmt - ein Richtwert bei Schwefelsäure als Elektrolyt liegt bei 1,2 nm Porenweite pro Volt angelegter Spannung. Unter Verwendung von Oxalsäure lassen sich dickere Filme erzeugen als unter Einsatz von Schwefelsäure. Bei der anodischen Oxidation nicht oxidiertes Aluminium auf der sogenannten Barriereseite kann anschließend in bekannter Weise in einem Säurebad abgelöst oder abgechliffen werden (siehe z.B. US-Patent 4,687,551), wobei nanoporöse Al₂O₃-Membranen mit einer geschlossenen (Barriereseite) und einer offenen (= Porenöffnungen) Oberfläche erhalten werden. Beim Abschleifen der Membran bis in den Bodenbereich der Poren werden zunächst Membranen mit einer offenen und einer halboffenen (= sehr kleine Porenöffnungen) Seite erhalten, bei weiterem Abschleifen sind Membranen mit beiderseitig durchgehenden, etwa gleich weiten Porenöffnungen erhältlich. Alternativ hierzu können durchgehende Poren auch durch Ätzen mit beispielsweise KOH in Glykol, wobei die Membran mit der Barriereseite auf das Ätzbad gelegt wird, erhalten werden.

Im Rahmen des erfindungsgemäßen Verfahrens wird unter anderem ein poröser Träger mit einem oder mehreren Precursor(en) aus der Gruppe der Verbindungen, welche die Verbindungen der Metalle aus den Nebengruppen Ib und VIIIb des Periodensystems, mit Ausnahme von Ru, umfaßt, beaufschlagt.

Diese Beaufschlagung oder Beladung kann dabei auf viele dem Fachmann an sich geläufigen Arten erfolgen, solange eine Immobilisierung der Metallverbindung(en) in Form des Metalls oder von Legierungen auf dem Träger möglich ist. So ist unter anderem die Abscheidung aus der Gasphase nach an sich bekannten CVD-Techniken möglich.

Eine bevorzugte Verfahrensmodifikation sieht vor, daß man den porösen Träger durch Tränken, Sprühen, Tauchen, Imprägnieren, Sprühtrocknen, Hicoaten oder Wirbelschichtcoaten, vorzugsweise durch Imprägnieren, mit der oder den Edelmetallverbindung(en) beaufschlagt.

Die Beladung des Trägers mit der oder den Edelmetallverbindung(en) kann in einem oder mehreren sequentiellen Schitten erfolgen, wobei zwischen einzelnen Fixierschritten gegebenenfalls Trocknungsphasen eingefügt werden können.

Als Aktivmetalle, die auf dem Träger, gegebenenfalls in einer Schale konzentriert werden können, sind alle reduzierbaren Metalle aus den Nebengruppen Ib und VIIIb des Periodensystems, insbesondere alle Edelmetalle hiervon, inklusive deren Mischungen, geeignet.

In bevorzugter Abwandlung des erfindungsgemäßen Verfahrens beaufschlagt man den Träger mit einer oder mehreren Verbindung(en) von Metallen aus der Gruppe, welche Kupfer, Silber, Gold, Eisen, Kobalt, Nickel, Rhodium, Osmium, Iridium, Palladium und Platin umfaßt.

Hiervon wiederum sind Verbindungen des Pd, Au, Pt, Ag, Rh, Cu, Ir, Ni und/oder Co bevorzugt. Besonders bevorzugt sind Verbindungen von Pd, Au, Pt, Ag und/oder Rh.

Noch eine zweckmäßige Variante des Verfahrens der Erfindung zeichnet sich dadurch aus, daß man den Träger mit einer oder mehreren Palladiumverbindung(en) allein oder einer oder mehreren Palladiumverbindung(en) zusammen mit einer oder mehreren Verbindung(en) von Metallen aus der Gruppe, welche Kupfer, Silber, Gold, Eisen, Kobalt, Nickel, Rhodium, Osmium, Iridium und Platin umfaßt, beaufschlagt.

Eine äußerst vorteilhafte Variante beinhaltet, daß man den Träger mit einer oder mehreren Palladiumverbindung(en) zusammen mit einer oder merheren Verbindung(en) des Goldes, beaufschlagt.

Das Verfahren der Erfindung beinhaltet als essentielle Maßnahme, daß man den mit wenigstens einem Precursor (Vorläuferverbindung des Aktivmetalls) beaufschlagten porösen Träger mit wenigstens einem Reduktionsmittel unter Erhalt von in den Poren des Trägers in situ hergestellten metallenen Nanopartikeln und/oder Legierungspartikeln behandelt.

Als Reduktionsmittel sind alle Verbindungen geeignet, die die eingesetzten MetallVerbindungen, vorzugsweise Salze, besonders bevorzugt Pd- und Au-Salze, zu den Metallen zu reduzieren vermögen.

In einer besonderen Verfahrensmodifikation setzt man ein oder mehrere Reduktionsmittel aus der Gruppe, welche Citrate wie Kaliumcitrat, Natriumcitrat, Ammoniumcitrat; Hydrazin, Hdroxylamin, Natriumhypophosphit, Alkaliborhydride wie Natriumborhydrid, Kaliumborhydrid; gasförmige Reduktionsmittel wie Wasserstoff, Kohlenmonoxid; Formaldehyd, Formiate, Acetate, Oxalate, geeignete Sulfanilate wie Hydroxymethansulfinsäure-Natriumsalz; und ein-oder zweiwertige Alkohole wie Ethanol, Ethylenglykol; umfaßt, ein.

Bevorzugt hiervon sind (Alkali/Erdalkali/Ammonium-)Citrate, Formiate, Acetate, Alkaliborhydride, Oxalate und geeignete Sulfanilate.

Eine vorteilige Ausführungsform der Erfindung setzt als Reduktionsmittel Ammoniumcitrat, Kaliumcitrat und/oder Natriumcitrat ein.

Besonders bevorzugt ist K-Citrat.

Das Reduktionsmittel wird im allgemeinen in stöchiometrischen Mengen bezogen auf die Metallverbindung(en), vorzugsweise aber in einem gewissen Überschuß eingesetzt. Der Überschuß kann beispielsweise 1,1 bis 2, vorzugsweise 1,1 bis 1,5 Molequivalente betragen.

Vorzugsweise wird das Verfahren der Erfindung so geführt, daß man die in-situ Reduktion bei Temperaturen zwischen Raumtemperatur und 150°C durchführt.

Im Rahmen der Erfindung werden die porösen Träger in einer besonders günstigen Variante unter Einsatz einer Lösung der Metallverbindung(en) damit beaufschlagt, beispielsweise wird durch Tränken oder Eintauchen in eine Lösung imprägniert.

Grundsätzlich kann diese Lösung eine Lösung der Metallverbindung(en) in einem wässrigen oder organischen Lösungsmittel sein. So kann man zur Beaufschlagung eine wäßrige Lösung, eine Lösung in einem organischen Lösungsmittel oder eine Mischung daraus einsetzen.

Als Lösungsmittel sind alle Verbindungen geeignet, in denen die gewählten Salze löslich sind und die nach der Imprägnierung leicht wieder durch Trocknung zu entfernen sind.

Besonders bevorzugt setzt man als Lösungsmittel Wasser ein. Hierbei ist die Natur und Reinheit des Wassers nur von untergeordneter Bedeutung. Man kann VE-Wasser, dest. Wasser oder Aqua bidest. einsetzen, ebenso wie in gewissem Maße auch Leitungswasser zum Einsatz kommen kann, solange die darin enthaltenen Stoffe die erfindungsgemäße Katalysatorherstellung nicht nachteilig beeinflußen.

Je nach Natur der zu lösenden Metallverbindung(en) kann die Natur von organischen Lösungsmitteln variieren.

Beispielsweise sind für Salze wie Acetate vor allem unsubstituierte Carbonsäuren, insbesondere Essigsäure geeignet. Für Chloride ist vor allem Wasser geeignet.

Die zusätzliche Verwendung eines weiteren Lösungsmittels ist dann zweckmäßig, wenn Salze in Essigsäure oder im Wasser nicht genügend löslich sind. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, Acetonitril, Dimethylformamid, aber auch Kohlenwasserstoffe wie Benzol.

Ferner kann man mit gutem Erfolg als organisches Lösungsmittel Methanol, Ethanol, Ethylenglykol, N-Methylpyrrolidon, Dimethylformamid, Dimethylacetamid und/oder Tetrahydrofuran oder eine Mischung dieser Stoffe mit Wasser einsetzen.

Die Verbindungen der edlen Metalle dienen als Precursor. D.h. es handelt sich um eine Vorstufe der Metalle, die durch Reduktion zum Metall umgesetzt werden kann. Es kann sich um ionische und nichtionische Verbindungen handeln.

Bei weitem bevorzugt sind die als Precursoren einzusetzenden Metallverbindungen Salze.

Als Salze sind alle Salze der Metalle geeignet, die löslich sind und keine für den Katalysator giftigen Bestandteile, wie z.B. Schwefel enthalten. Bevorzugt sind die Acetate und die Chloride.

Für den Fall, daß man Palladium-Precursorverbindungen einsetzt, sind lösliche Palladiumverbindungen, insbesondere wasserlösliche Salze, aus einer Gruppe, welche Palladium(II)-acetat, Palladium(II)-chlorid, Palladium(II)-nitrat und Tetrachloropalladium(II)säure-Natriumsalz [Na₂PdCl₄] umfaßt, bevorzugt.

Im Falle der Chloride sind PdCl₂ und Na₂PdCl₄ besonders bevorzugte Presursoren.

Weitere bevorzugt eingesetzte lösliche Metallverbindungen, insbesondere wasserlösliche Salze, sind Tetrachlorogold(III)säure [HAuCl₄], Gold(III)acetat [Au(OAc)₃], Kaliumaurat [KAuO₂], Hexachloroplatin(IV)-säure-Hydrat, Hexachloroiridium(IV)-säure-Hydrat und/oder Rhodium(III)-chlorid-Hydrat.

Dabei muß generall im Falle der Chloride sichergestellt werden, daß die Chloridionen vor dem Einsatz des Katalysators entfernt werden. Dies geschieht durch Auswaschen des dotierten Trägers, z.B. mit Wasser, nachdem die Metalle durch Reduktion zu den metallischen Nanopartikeln auf dem Träger fixiert wurden.

Die Metallverbindungen werden üblicherweise in Konzentrationen von etwa 0,1 bis 100 g pro Liter, vorzugsweise von 1 bis 50 g pro Liter bezogen auf das Lösemittel eingesetzt.

Obwohl auch bereits ohne weitere Zusätze stabile Katalysatoren mit Nanopartikeln auf einem Träger erhältlich sind, ist es dennoch beim Verfahren der Erfindung bei weitem bevorzugt, die Beaufschlagung des porösen, vorzugsweise nanoporösen, Trägers und/oder die Reduktion des beaufschlagten Trägers in Gegenwart eines Kolloid-Stabilisators oder mehrerer Koiloid-Stabilisatoren auszuführen.

Als Stabilisatoren sind alle Verbindungen geeignet, die die durch Reduktion erhaltenen Nanoteilchen durch Umhüllung zu komplexieren vermögen und dadurch das weitere Wachstum und die Agglomeration der Nanoteilchen zu verhindern vermögen.

Zu im Rahmen der Erfindung einsetzbaren Stabilisatoren gehören unter anderem Betaine, Tenside, Polymere wie Polyvinylpyrrolidon (PVP), Polyvinylalkohol (PVA), Polyacrylamid (PAA), Polyelektrolyte, Citrate, substituierte Phosphine, substituierte Sulfanilsäuren, Chloride, Aminosäuren oder deren Mischungen. Man kann unter anderem auch Copolymere einsetzen, die aus Monomeren mit Betain-Gruppen sowie weiteren Monomeren wie beispielsweise Acrylsäure, Acrylsäureester, Acrylsäureamide, Carbonsäurevinylester, Vinyl-alkylether, N-Vinylpyridin, N-Vinylpyrrolidon, N-Vinylcarbonsäureamide aufgebaut sind.

Eine exponierte Verfahrensabwandlung zeichnet sich dadurch aus, daß man als Kolloid-Stabilisator eine oder mehrere Verbindung(en) aus der Gruppen, welche Betaine, PVP, Phosphine, Citrate, Oxalate, Formiate, Acetate, Sulfanilate, PVA und PAA umfaßt, zusetzt.

Bevorzugt sind Betaine, PVP, PVA, Citrate, substituierte Phosphine, substituierte Sulfanilsäuren und/oder Chloride.

Besonders bevorzugt sind K-Citrat, Ammonium-Citrat, PVP K 30, Dimethyldodecylammoniumpropansulfonat.

Für das erfindungsgemäße Verfahren werden die Stabilisatoren üblicherweise in Mengen von 5 bis 1000 Gew.-% bezogen auf das oder die Metalle eingesetzt.

Die Zugabe des Stabilisators kann in beliebiger Reihenfolge stattfinden. Der Stabilisator kann zur Metallverbindung gegeben werden, mit welcher der Träger imprägniert wird. Der Träger kann zuerst mit dem Kolloidstabilisator getränkt werden. Der imprägnierte Träger kann mit dem Kolloid-Stabilisator in Kontakt gebracht werden. Der oder die Kolloid-Stabilisatoren können auch mit dem Reduktionsmittel zusammen angewandt werden. Auch eine nachträgliche Stabilisierung (nach Reduktion) ist unter Umständen möglich.

Eine ganz besondere Variante der Erfindung sieht vor, daß man ein oder mehrere Verbindungen einsetzt, die als Kolloid-Stabilisator und zugleich als Reduktionsmittel wirken. Dies bedeutet, daß Reduktionsmittel und Stabilisator auch identisch sein können. So wirkt K-Citrat bei Pd/Au beispielsweise sowohl als Reduktionsmittel als auch als Stabilisator.

Es ist mithin im Rahmen der Erfindung bevorzugt als Reduktionsmittel und Kolloid-Stabilisator NH₄-, K- und/oder Na-Citrat einzusetzen.

Ganz besonders zweckmäßig ist Kaliumcitrat.

Der Stabilisator kann nach der Trägerfixierung auf den Nanoteilchen verbleiben oder ggf. entfernt werden, falls die Gegenwart des Stabilisators für die Katalyse stören sollte. Die vollständige oder teilweise Entfernung des Stabilisators kann bei Bedarf z. B. hydrolytisch mit einem Lösungsmittel, thermisch oder oxidativ, z. B. durch Abbrennen in Luft bei 300 bis 500 °C erfolgen, sowohl vor dem Einbau des Katalysators in den Reaktor als auch in situ im Reaktor.

Die Beaufschlagung des Trägers mit Metallverbindung(en) und deren Reduktion können nacheinander in zwei Schritten durchgeführt werden oder in einem "Eintopfverfahren".

In einer Variante kann es bevorzugt sein, daß man den ersten Schritt und den zweiten Schritt nacheinander ausführt. Dies gestattet vorteilig, daß man den mit wenigstens einer Metallverbindung beaufschlagten porösen, vorzugsweise nanoporösen, Träger vor der Reduktion einem Trocknungsschritt unterwirft. Auf diese Weise lassen sich zum Beispiel durch mehrfache Wiederholung der Imprägnierung und Trocknung verschiedene "Schalen" von Metallverbindungen auf den Träger bringen.

Alternativ hierzu ist es auch bevorzugt, daß man den ersten Schritt und den zweiten Schritt in einem Eintopfverfahren ohne Isolierung, Reinigung oder Trocknung des beaufschlagten porösen, vorzugsweise nanoporösen Trägers durchführt.

Im Sinne einer bevorzugten Ausführungsform der Erfindung wird ein Träger zunächst mit im wesentlichen wässrigen Salzlösungen von reduzierbaren Aktivmetallen vorimprägniert, wobei diese Vorimprägnierung nicht zu einer Schale führen muß, d. h. der Träger wird unter Umständen "durchimprägniert".

Es kann jedoch auch eine Schale und damit ein Schalenkatalysator erzeugt werden. Dies geschieht zum Beispiel durch bestimmte nicht vollständige Imprägnierung des Trägers und/oder durch geeignete Führung der Reduktion.

Durch Imprägnierung und anschließende, ggf. nach einem Trocknungsschritt, Behandlung mit einem Reduktionsmittel unter solchen Bedingungen (Konzentration, Temperatur, Zeit etc.) und ggf. in Gegenwart von Stabilisatoren, werden die Aktivmetalle zu den Metallen in der Oxidationsstufe 0 reduziert und können sich als Nanoteilchen in einer Schale des Trägerformkörpers vom eggshell- oder eggwhite-Typ anreichern.

Vorzugsweise führt man also die Beaufschlagung und/oder Reduktion so, daß die Metallverbindungen in den Poren des Trägers in einer schalenförmigen oberflächennahen Zone zu den entsprechenden Metallen bzw. Legierungen in Form von ggf. stabilisierten Nanopartikeln unter Erhalt eines Schalenkatalysators reduziert werden.

Gasförmige Reduktionsmittel wie H₂ oder CO oder Ethylen können allerdings nur verwendet werden, wenn bei der Imprägnierung mit den Metallsalzen bereits eine Schalenstruktur erzeugt worden ist.

Für den Mechanismus der Schalenbildung bei der Herstellung von Edelmetall-Schalenkatalysatoren auf porösen Keramikträger-Formkörpern kann - ohne hierdurch die Erfindung auf den Mechanismus einschränken zu wollen - angenommen werden, daß an der inneren Grenzfläche Aktivmetallsalz/Reduktionsmittel die rasche Reduktion zu den Nanoteilchen stattfindet, diese aufgrund ihrer Größe (inklusive der Umhüllung mit Stabilisator) in der äußeren Schale immobilisiert werden, und weiteres Aktivmetallsalz aus den inneren Bereichen des Formkörpers in Richtung Oberfläche nachdiffundieren, um innerhalb der Schale nach Erreichen der langsam nach innen fortschreitenden Reduktionsmittelfront ebenfalls reduziert und auf dem Träger abgeschieden zu werden.

Ein wesentlicher Vorteil der Erfindung besteht unter anderem darin, daß besonders stabile Schalen auch von größerer Dicke erzeugt werden können. Bevorzugt erhält man eine Schalendicke im Bereich zwischen 5 um und 5000 µm.

Zweckmäßig werden Schalenkatalysatoren erhalten, die in den Poren Nanopartikel mit einem mittleren Teilchendurchmesser im Bereich von 1 bis 100 nm aufweisen, ebenso wie in der Schale. D. h., die Partikel der Schale agglomeriern nicht oder nur wenig.

Der Träger kann vor, während und/oder nach der in situ Generierung der Nanopartikel noch mit weiteren Aktivatoren, insbesondere Alkali-Acetaten, und gegebenenfalls Promotoren, beispielsweise Zr-, Ti-, Cd-, Cu-, Ba- und/oder Re-Verbindungen beladen werden.

Eine besonders interessante Verfahrensabwandlung umfaßt daher, daß man nach, vor oder während der Beaufschlagung und/oder Reduktion ein oder mehrere Aktivatoren und/oder Promotoren aufbringt.

Einige bevorzugt gemäß der Erfindung herstellbare Katalysatorsysteme, vorzugsweise Schalenkatalysatoren, enthalten z. B. neben Palladium und Gold auch noch K-Acetat als Aktivator und/oder Cadmium oder Bariumverbindungen als Promotoren.

Die Metallgehalte von besonders bevorzugten Katalysatoren haben folgende Werte:

Der Pd-Gehalt der Pd/K/Cd- und der Pd/K/Ba-Katalysatoren beträgt im allgemeinen 0.6 bis 3.5 Gew.-%, vorzugsweise 0.8 bis 3.0 Gew.-%, insbesondere 1.0 bis 2.5 Gew.-%.

Der Pd-Gehalt der Pd/Au/K-Katalysatoren beträgt im allgemeinen 0.5 bis 2.0 Gew.-%, vorzugsweise 0.6 bis 1.5 Gew.-%.

Der K-Gehalt aller drei Kyatalysator-Arten beträgt im allgemeinen 0.5 bis 4.0 Gew.-%, vorzugsweise 1.5 bis 3.0 Gew.-%.

Der Cd-Gehalt der Pd/K/Cd-Katalysatoren beträgt im allgemeinen 0.1 bis 2.5 Gew.-%, vorzugsweise 0.4 bis 2.0 Gew.-%.

Der Ba-Gehalt der Pd/K/Ba-Katalysatoren beträgt im allgemeinen 0.1 bis 2.0 Gew.-%, vorzugsweise 0.2 bis 1.0 Gew.-%.

Der Au-Gehalt der Pd/K/Au-Katalysatoren beträgt im allgemeinen 0.2 bis 1.0 Gew.-%, vorzugsweise 0.3 bis 0.8 Gew.-%.

Von jedem der auf die Trägerteilchen aufzubringenden Elemente (beispielsweise Pd/K/Au, Pd/K/Cd, Pd/K/Ba) muß mindestens ein Salz aufgebracht werden. Man kann mehrere Salze eines Elements aufbringen, aber im allgemeinen bringt man von jedem der drei Elemente genau ein Salz auf. Die notwendigen Salzmengen können in einem Schritt oder durch Mehrfachimprägnierung aufgebracht werden. Die Salze können nach bekannten Methoden wie Tränken, Aufsprühen, Aufdampfen, Tauchen oder Ausfällen auf den Träger aufgebracht werden.

Nach der erfindungsgemäßen Methode werden natürlich nur die Edelmetallsalze, also Pd und Au, zu den entsprechenden Edelmetall-Nanoteilchen reduziert und nicht die "unedlen" Bestandteile K, Cd, Ba. Letztere können zusammen mit den Edelmetallsalzen oder auch vorher oder nachher auf den Träger aufgebracht werden.

Normalerweise wird nach der erfindungsgemäßen Methode zuerst eine Schale aus Pd/Au erzeugt und danach mit K-Acetat-Lösung nachgetränkt, wobei das K gleichmäßig über den Pelletquerschnitt verteilt ist.

Sollen mehrere Edelmetalle auf dem Träger fixiert werden (z.B. Pd und Au), können nach der erfindungsgemäßen Methode Legierungen oder strukturierte Nanostrukturen, d. h. Gold auf Palladium oder Palladium auf Gold hergestellt werden.

Die nach dem Verfahren der Erfindung hergestellten Schalenkatalysatoren können für eine Vielzahl von heterogen katalysierten Reaktionen eingesetzt werden.

Hierzu gehören unter anderem Aminierungen, Hydrierungen, Dehydrierungen, Dehydrocyclisierungen, Hydroxylierungen, Oxidationen, Epoxidierungen, Skelettisomerisierungen sowie Kombinationen dieser Reaktionstypen, zur gezielten Umsetzung organischer Moleküle.

Die imprägnierten und reduzierten Formkörper lassen sich - insbesondere nach einer Aktivierung - als Schalenkatalysatoren für Hydrierungs-. Oxidations- und Isomerisierungsreaktionen, ganz besonders für Selektivhydrierungsreaktionen und Partialoxidationen einsetzen.

Beispiele hierfür sind unter anderem: Selektivhydrierung von Piopin, Selektivhydrierung von Butadien, Selektivhydrierung von Acetylen, Selektivhydrierung von Butinol, Selektivhydrierung von Octadien zu Octen, Selektivhydrierung von Benzol zu Cyclohexen, Hydrierung von Kohlenmonoxid, Hydrierung von Kohlendioxid, Hydrierung von Maleinsäureanhydrid, Hydrierung von NOₓ zu NH₃ oder NH₂OH, Carbonsäureamide aus Nitrilen, Amine aus Carbonsäuren, Aminierung von Aromaten, insbesondere Reaktion von Benzol mit Ammoniak zu Anilin, reduktive Aminierung von Aldehyden und Ketonen zu Aminen, Wacker-Synthese, Acetaldehyd auf basis Ethylen, Oxidation von Butan zu Maleinsäureanhydrid, Oxidation von Kohlenmonoxid, Oxidation von Alkoholen zu Aldehyden, Ketonen oder Carbonsäuren, Oxidationen von Alkanen zu Alkoholen, Aldehyden und Ketonen, Oxidationen von Aldehyden und Ketonen zu Carbonsäuren, Hydroxylierung von Aromaten, z.B. Oxidation von Benzol zu Phenol oder Toluol zu Kresol, Oxidation von Propylen zu Acrolein bzw. Acrylsäure, Ammonoxidation z.B. von Toluol zu Benzonitril oder von Propylen zu Acrylnitril, Epoxide lassen sich in Aldehyde/Ketone und unter hydrierenden Bedingungen in Alkohole umwandeln, z. B. Styroloxid-Derivate zu den entsprechenden Phenylacetaldehyden bzw. unter hydrierenden Bedingungen zu Phenylethanolen.

Auf erfindungsgemäße Weise hergestellte Pd/Au-Schalenkatalysatoren können bevorzugt in der Vinylacetat-Synthese zur Herstellung von Vinylacetat in der Gasphase aus Ethylen, Essigsäure und Sauerstoff oder Sauerstoff enthaltenden Gasen zum Einsatz kommen.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Bezüglich der Synthese von Vinylacetat (VAM) hat es sich erwiesen, daß die für die Synthese aus Ethylen, Essigsäure und Sauerstoff verwendeten Trägerkatalysatoren bevorzugt Pd enthalten und ein Alkalielement, vorzugsweise K. Als weitere Zusätze werden mit Erfolg Cd, Au oder Ba verwendet.

Die Metallsalze können durch Tränken, Aufsprühen, Aufdampfen, Tauchen oder Ausfällen auf den Träger aufgebracht werden.

Bei den Pd/Au/K-Katalysatoren hat es sich als vorteilhaft erwiesen, die beiden Edelmetalle in Form einer Schale auf den Träger aufzubringen, d.h. die Edelmetalle sind nur in einer oberflächennahen Zone verteilt, während die weiter innen liegenden Bereiche des Trägerformkörpers nahezu edelmetallfrei sind. Die Schichtdicke dieser katalytisch aktiven Schalen beträgt ca. 0.1-2 mm.

Mit Hilfe von Schalenkatalysatoren ist eine selektivere Verfahrensdurchführung möglich als mit Katalysatoren, bei denen die Trägerteilchen bis in den Kern imprägniert ("durchimprägniert") sind, bzw. eine Kapazitätserweiterung.

Dabei bietet es sich an. die Reaktionsbedingungen gegenüber den durchimprägnierten Katalysatoren unverändert zu halten und mehr Vinylacetat pro Reaktorvolumen und Zeit herzustellen. Dadurch wird die Aufarbeitung des erhaltenen Rohvinylacetats erleichtert, da der Vinylacetatgehalt im Reaktorausgangsgas höher ist, was weiterhin zu einer Energieersparnis im Aufarbeitungsteil führt. Geeignete Aufarbeitungen werden z.B. in US 5 066 365, DE 34 22 575, DE 34 08 239, DE 29 45 913, DE 26 10 624, US 3 840 590 beschrieben. Hält man dagegen die Anlagenkapazität konstant, so kann man die Reaktionstemperatur senken und dadurch bei gleicher Gesamtleistung die Reaktion selektiver durchführen, wobei Edukte eingespart werden. Dabei wird auch die Menge des als Nebenprodukt entstehenden und daher auszuschleusenden Kohlendioxids und der mit dieser Ausschleusung verbundene Verlust an mitgeschlepptem Ethylen geringer. Darüberhinaus führt diese Fahrweise zu einer Verlängerung der Katalysatorstandzeit.

Die Erfindung liefert hierfür ein einstufiges Herstellverfahren für Sol-beschichtete Trägerkatalysatoren, indem die Sole in-situ in den Poren des Trägers durch Reduktion hergestellt werden, d.h. Herstellung der Sole und Trägerfixierung simultan in einem Schritt erfolgen. Auf diese Weise kann die Schalendicke den Erfordernissen leichter angepaßt werden, insbesondere sind größere Schalendicken möglich als bei der Soltränktechnik, bei der die Diffusion der Sole von außen in die Trägerporen auch durch den mechanischem Siebeffekt behindert ist. Ferner sind höhere Edelmetallbeladungen auf dem Träger möglich, es werden Arbeitsschritte eingespart und das energieintensive Handling mit hochverdünnten Lösungen wird vermieden. Gegebenenfalls kann im Falle wohldefinierter Porenstrukturen der Träger über die Porengröße des Trägers die Kolloidgröße exakt eingestellt werden, so daß monomodale Verteilungen von Kolloiden einfacher hergestellt werden können. Die zur Präparation von Solen benötigten peinlichst sauberen Apparaturen und Lösungsmittel (bidest. Wasser) entfallen bei der in-situ Herstellung vollkommen. Verunreinigungen in Solen führen zu größeren Teilchengrößen sowie zur Agglomeration von Teilchen.

Eine schalenförmige Beladung mit Metallsalzen bei der Vorimprägnierung nach bekannten Techniken sowie ein rascher Wasserentzug beim Trocknen z.B. im Vakuum fördern zusätzlich zur erfindungsgemäßen Reduktionsmethode die Ausbildung von Schalen bzw. erlauben eine weitere Verringerung der Schalendicke, falls dies erwünscht ist.

Die erfindungsgemäßen Katalysatoren weisen eine wesentlich einheitlichere Aktivmetall-Verteilung und höhere Edelmetall-Dispersion auf als konventionell hergestellte VAM-Katalysatoren. Die hohe Dispersion bleibt auch im Dauerbetrieb aufgrund verminderter Agglomeration der Edelmetallteilchen weitgehend erhalten, woduch die Deaktivierung der erfindungsgemäßen Katalysatoren verlangsamt wird und lange Standzeiten resultieren. Die erfindungsgemäße Herstellung führt vorteilhafterweise zu einer im wesentlichen monomodalen und sehr schmalbandigen Partikelgrößenverteilung.

Ferner sind die mittleren Edelmetall-Partikeldurchmesser wesentlich geringer als bei herkömmlichen Katalysatoren. Dies hat eine hohe Aktivmetalloberfläche und damit hohe katalytische Aktivität zur Folge.

Die nachfolgenden Beispiele dienen zur eingehenderen Erläuterung und Veranschaulichung der Erfindung, ohne daß diese darauf eingeschränkt sein soll.

### Beispiel 1

200g SiO₂ -Träger (Siliperl AF125, Firma Engelhard) mit einer BET-Oberfläche von 300 m²/g wurde mit einer salzsauren Lösung von 3.33 g (18.8 mmol) Palladiumchlorid und 1,85 g (4.7 mmol) Goldsäure in 500 ml Wasser bei einer Temperatur von 30-32°C diskontinuierlich in einer Dragieranlage über 35 min besprüht. Anschließend wurden die Trägerkugeln getrocknet und mit 20 g Trikaliumcitrat-Hydrat in 200 ml Wasser gelöst über 25 min besprüht. Bei einer Trommelumdrehung von 10 U/min wurde mit 1 bar diskontinuierlich aufgesprüht. Die Eingangstemperatur (Föhntemperatur) war 60°C und die Produkttemperatur war zwischen 32-30°C. Man erhielt einen homogen imprägnierten Schalen-Katalysator mit einer Schalendicke von 400 µm.

Mittels TEM wurde der Duchmesser der Nanopartikel bestimmt. Der mittlere Teilchendurchmesser beträgt 30 nm.

### Beispiel 2

20g des gleichen Trägers wie in Beispiel 1 wurden mit einer Lösung von 335 mg Palladiumchlorid und 186 mg Goldsäure durch Tränkung imprägniert und getrocknet. Bei 65° C wurde mit 1,52 g Trinatriumcitrat-Dihydrat in 19,6 ml Wasser getränkt und nach drei-stündigem Stehen bei 65° C getrocknet.

Nach Durchschneiden einer repräsentativen Anzahl von Pellets wurde die Schalendicke mittels Lichtmikroskopie und XPS-Lins-Scans ausgemessen.

Die Schalendicke beträgt 1 mm.

Mittels TEM wurde der Durchmesser der Nanopartikel bestimmt. Der mittlere Teilchendurchmesser beträgt 40 nm.

### Beispiel 3

20 g SiO₂-Träger (Typ Aerosil 200, Firma Degussa) wurden mit einer Lösung (19.6 ml) von 325 mg (1.89 mmol) Palladiumchlorid und 189 mg (0.473 mmol) Goldsäure durch Tränkung imprägniert und getrocknet. Der Träger wurde mit 19 ml Wasser durch Tränkung benäßt und mit 1.68 g Trikaliumcitrat in 10 ml Wasser getränkt und getrocknet.

Nach Durchschneiden einer repräsentativen Anzahl von Pellets wurde die Schalendicke mittels REM ausgemessen.

Die Schalendicke beträgt 140 µm.

Mittels TEM wurde der Durchmesser der Nanopartikel bestimmt. Die mittlere Teilchengröße beträgt 60 nm.

### Beispiel 4

Palladiumchlorid (335 mg) und Goldsäure (186 mg) wurden in Wasser (19.6 ml) gelöst und durch Tränkung auf SiO2-Träger vom Typ Siliperl AF 125 (10.0 g) gebracht. Der Träger wurde getrocknet und mit einer wässrigen Lösung aus Kaliumformiat (0.5 g) und Sulfanilsäure-Natriumsalz (0.2 g) getränkt und getrocknet. Das Pd/Au-Verhältnis beträgt Pd:Au=8:2.

### Beispiel 5

186 mg Goldsäure wurde auf Siliperl AF 125 durch Tränkung imprägniert, bei 120°C getrocknet und mit Citratlösung (19.6 ml) reduziert. Nach 12 h wurden die dunkelgrauen Perlen getrocknet, um anschließend mit 16 ml einer 60°C warmen essigsauren Palladiumacetat-Lösung ( 424 mg, 1,89 mmol) getränkt zu werden. Die Trocknung erfolgte im Vakuumtrockenschrank, wo bei 120°C das Pd-Salz thermisch reduziert wurde.

Mittels TEM wurde der Teilchendurchmesser der Nanoteilchen bestimmt. Der mittlere Durchmesser beträgt 20 nm.

### Beispiel 6:

325 mg Palladiumchlorid und 186 mg Goldsäure wurden in Wasser gelöst. 20 g Träger (Siliperl AF 125) wurde in einem Rundkolben (250 ml) vorgelegt und mit Goldsäure- und Palladiumchlorid-Lösung (19.6 ml) getränkt. Anschließend trocknete man den Träger bei 120°C 4 h lang. Man tränkte mit einer viskosen Kaliumcitratlösung (10 ml), schüttelte gut und trocknete nochmals.

Die Schale ist 75µm dick und ist schwarz.

### Beispiel 7:

Goldsäure (189 mg, 0.473 mmol) und Palladiumchlorid (325 mg, 1.89 mmol) wurden in Wasser gelöst. Der SiO₂-Träger (20.0 g; Typ D11-10, Firma BASF) wurde mit der Lösung getränkt und anschließend bei 120°C 5 h getrocknet. Nach dem Erkalten wurde zu den verschiedenen Trägern Wasser (A = 16 ml, D = 19 ml) gegeben, um schließlich eine Trikaliumcitrat-Lösung (1.68 g in 10 ml) eindiffundieren zu lassen. Während der Wasserzugabe entfärbte sich der Träger von beige nach weiß. Der Träger wurde bei 120°C 5 h getrocknet.

### Reaktortests:

Die in den Beispielen und Vergleichsbeispielen hergestellten Katalysatoren werden in einem Mikrofestbettrohrreaktor mit einem Füllvolumen von 36 ml getestet. Die Gasdosierung erfolgt über MFC's, die Essigsäure wird mit einem LFC (Fa. Bronkhorst) dosiert. Die Mischung der Gase und der Essigsäure erfolgt in einem mit Füllkörpern beschickten Gasmischrohr. Der Reaktoraustrag wird auf Normaldruck entspannt und durch einen Glaskühler geleitet. Das aufgefangene Kondensat wird off-line mit GC analysiert. Die nichtkondensierbaren Gase werden durch on-line GC quantitativ erfaßt.

Vor der Messung wird der Katalysator im Reaktor wie folgt aktiviert:

Der Katalysator wird unter N₂ bei Normaldruck von ca. 25°C auf 155°C aufgeheizt.

Zugleich wird die Gastemperatur auf 150°C und die Gasmischtemperatur auf 160°C erhöht. Die Bedingungen werden einige Zeit gehalten.

Anschließend wird Ethylen zugeführt und der Druck auf 10 bar erhöht. Nach einer Haltezeit wird Essigsäure zudosiert und die Bedingungen für einige Zeit gehalten.

Nach der Aktivierung wird der Katalysator wie folgt angefahren und vermessen:

Sauerstoff wird nach dem Gasmischrohr zugegeben und die Sauerstoffkonzentration schrittweise auf 4.8 Vol.-% (1. Messung) und später auf 5.2 Vol.-% (2. Messung) erhöht. Es ist immer darauf zu achten, daß die Explosionsgrenzen des zündfähigen Ethylen/O₂-Gemisches nicht überschritten werden. Gleichzeitig wird die Reaktortemperatur auf 170°C erhöht.

Die Reaktion wird ständig mit dem Gaschromatographen überwacht.

Bei gleichmäßiger Reaktion, d. h. bei konstanter Reaktortemperatur und gleichbleibender Konzentration von Vinylacetat und CO₂ im Produktgasstrom, beginnt die Probennahme.

Über eine Dauer von ca. 1 h wird eine Flüssigprobe und mehrere Gasproben entnommen.

Der Produktgasstrom wird mit einer Gasuhr bestimmt. Nach Beendigung der Austestung wird zunächst die Sauerstoffkonzentration schrittweise zurückgefahren.

Die Zusammensetzung der verwendeten Katalysatoren findet sich in Tabelle 1. Die erhaltenen Reaktorergebnisse finden sich in Tabelle 2.

**Tabelle 1**

| Katalysatordaten | | | | |
|---|---|---|---|---|
| Bsp./ Vgl. | Precursoren | Reduktionsmittel | Stabili-sator | Schalen Dicke [µm] |
| 2 | PdCl₂, HAuCl₄ | Na-citrat | - | 1000 |
| 3 | PdCl₂, HAuCl₄ | K-citrat | - | 140 |
| 4 | PdCl₂, HAuCl₄ | K-formiat | Na-sulfanilsäure | 250 |
| 5 | HAuCl₄, Pd(OAc)₂ | K-citrat | - | 200 |
| 6 | PdCl₂, HAuCl₄ | K-citrat (viskos) | - | 75 |
| 7 | PdCl₂, HAuCl₄ | K-citrat | - | 150 |

**Tabelle 2**

| Katalysatorwirkung im Mikroreaktor | | | | | | |
|---|---|---|---|---|---|---|
| Bsp./ Vgl. | Zusammensetzung | Träger | O₂ Feed Konz. [%] | Coating Methode | Selektivität [%] | STY [g/l*h] |
| 2 | Pd/Au=4:1 | Siliperl | 4.8 | Impregnierung | 90.3 | 93 |
| 3 | Pd/Au=4:1 | Aerosil 200 | 4.8 | Impregnierung | 90.7 | 110 |
| 4 | Pd/Au=4:1 | Siliperl | 4.8 | Impregnierung | 86.3 | 49 |
| 5 | Pd/Au=4:1 | Siliperl | 4.8 | Impregnierung | 92.0 | 147 |
| 6 | Pd/Au=4:1 | Siliperl | 4.8 | Impregnierung | 89.7 | 110 |
| 7 | Pd/Au=4:1 | D11-10 | 4.8 | Impregnierung | 88.9 | 70 |

Weitere Vorteile und Ausführungsformen der Erfindung ergeben sich aus den nachfolgenden Patentansprüchen.

## Patentansprüche

1. Verfahren zur Herstellung eines auf porösen Trägerteilchen ein oder mehrere Metalle aus der Gruppe von Metallen, welche die Nebengruppen Ib und VIIIb des periodischen Systems der Elemente umfaßt, aufweisenden Katalysators, dadurch gekennzeichnet, daß man in einem ersten Schritt einen porösen Träger mit mindestens einer Lösung von mindestens einer Metallverbindung aus der Gruppe der Metalle der Nebengruppen Ib und VIIIb des Periodensystems, mit der Ausnahme von Ruthenium, als einem oder mehreren Precursor(en) aus der Gruppe dieser Metalle beaufschlagt und daß man in einem zweiten Schritt den mit wenigstens einem Precursor beaufschlagten porösen Träger mit wenigstens einem Reduktionsmittel derart behandelt, so daß durch Reduktion der Metallverbindung ein Sol in den Poren des Trägers erzeugt wird und in diesem Schritt auf dem Träger fixiert wird.

2. Verfahren nach Anspruch 1, dadurch gekennzeichnet, daß man einen inerten, porösen, vorzugsweise nanoporösen, Träger aus Siliciumdioxid, Aluminiumoxid, Titandioxid, Zirkondioxid, Oxidgemischen der genannten Verbindungen, Mischoxiden der genannten Verbindungen und/oder Aluminiumsilikaten in Form von Pulvern, Folien, Bändern, Membranen, Strängen, Platten, Tabletten, Wagenrädern, Monolithen, Kugeln, Splittern, Ringen, Voll-Extrudaten, Hohl-Extrudaten oder Sternen einsetzt.

3. Verfahren nach Anspruch 1 oder 2, dadurch gekennzeichnet, daß man den porösen Träger durch Tränken, Sprühen, Tauchen, Imprägnieren, Sprühtrocknen, Hicoaten oder Wirbelschichtcoaten, vorzugsweise durch Imprägnieren, mit der oder den Metallverbindung(en) beaufschlagt.

4. Verfahren nach einem oder mehreren der vorhergehenden Ansprüche, dadurch gekennzeichnet, daß man den Träger mit einer oder mehreren Verbindung(en) von Metallen aus der Gruppe, welche Kupfer, Silber, Gold, Eisen, Kobalt, Nickel, Rhodium, Osmium, Iridium, Palladium und Platin umfaßt, beaufschlagt.

5. Verfahren nach einem oder mehreren der vorhergehenden Ansprüche, dadurch gekennzeichnet, daß man den porösen Träger mit einer oder mehreren Palladiumverbindung(en) allein oder einer oder mehreren Palladiumverbindung(en) zusammen mit einer oder mehreren Verbindung(en) von Metallen aus der Gruppe, welche Kupfer, Silber, Gold, Eisen, Kobalt, Nickel, Rhodium, Osmium, Iridium und Platin umfaßt, beaufschlagt.

6. Verfahren nach einem oder mehreren der vorhergehenden Ansprüche, dadurch gekennzeichnet, daß man den porösen Träger mit einer oder mehreren Palladiumverbindung(en) zusammen mit einer oder mehreren Verbindung(en) des Goldes, beaufschlagt.

7. Verfahren nach einem oder mehreren der vorhergehenden Ansprüche, dadurch gekennzeichnet, daß man ein oder mehrere Reduktionsmittel aus der Gruppe, welche Citrate wie Kaliumcitrat, Nariumcitrat, Ammoniumcitrat; Hydrazin, Hydroxylamin, Natriumhypophosphit, Alkaliborhydride wie Natriumborhydrid, Kaliumborhydrid; gasförmige Reduktionsmittel wie Wasserstoff, Kohlenmonoxid; Formaldehyd, Formiate, Acetate, Oxalate, Sulfanilate wie Hydroxymethansulfinsäure-Natriumsalz; und ein- oder zweiwertige Alkohole wie Ethanol, Ethylengykol; umfaßt, einsetzt.

8. Verfahren nach einem doer mehreren der vorhergehenden Ansprüche, dadurch gekennzeichnet, daß man als Reduktionsmittel Kaliumcitrat, Natriumcitrat und/oder Ammoniumcitrat einsetzt.

9. Verfahren nach einem oder mehreren der vorhergehenden Ansprüche, dadurch gekennzeichnet, daß man den porösen, vorzugsweise nanoporösen, Träger unter Einsatz einer Lösung der Metallverbindung(en) damit beaufschlagt.

10. Verfahren nach Anspruch 9, dadurch gekennzeichnet, daß man zur Beaufschlagung eine wäßrige Lösung, eine Lösung in einem organischen Lösungsmittel oder eine Mischung daraus einsetzt.

11. Verfahren nach Anspruch 10, dadurch gekennzeichnet, daß man als Lösungsmittel Wasser einsetzt.

12. Verfahren nach Anspruch 10, dadurch gekennzeichnet, daß man als organisches Lösungsmittel Methanol, Ethanol, Ethylenglykol, N-Methylpyrrolidon, Dimethylformamid, Dimethylacetamid und/oder Tetrahydrofuran oder eine Mischung eines oder mehrerer dieser Stoffe mit Wasser einsetzt.

13. Verfahren nach einem oder mehreren der vorhergehenden Ansprüche 9 bis 12, dadurch gekennzeichnet, daß man lösliche Palladiumverbindungen, insbesondere wasserlösliche Salze, aus einer Gruppe von Pd-Precursoren, welche Palladium(II)-acetat, Palladium(II)-chlorid, Palladium(II)-nitrat und Tetrachloropalladium(ll)säure-Natriumsalz [Na₂PdCl₄] umfaßt, einsetzt.

14. Verfahren nach einem oder mehreren der vorhergehenden Ansprüche 9 bis 13, dadurch gekennzeichnet, daß man lösliche Metallverbindungen, insbesondere wasserlösliche Salze, aus einer Gruppe von Metall-Precursoren, welche Tetrachlorogold(III)säure, Gold(III)acetat [Au(OAc)₃], Kaliumaurat [KAuO₂], Hexachloroplatin(IV)-säure-Hydrat, Hexachloroiridium(IV)-säure-Hydrat und Rhodium-(III)-chlorid-Hydrat umfaßt, einsetzt.

15. Verfahren nach einem oder mehreren der vorhergehenden Ansprüche, dadurch gekennzeichnet, daß man die Beaufschlagung des porösen, vorzugsweise nanoporösen Trägers und/oder die Reduktion des beaufschlagten Trägers in Gegenwart eines Kolloid-Stabilisators oder mehrerer Kolloid-Stabilisatoren ausführt.

16. Verfahren nach Anspruch 15, dadurch gekennzeichnet, daß man als Kolloid-Stabilisator eine oder mehrere Verbindung(en) aus der Gruppen, welche Betaine, PVP, Citrate, Oxalate, Formiate, Acetate, Sulfanilate, PVA und PAA umfaßt, zusetzt.

17. Verfahren nach Anspruch 15 oder 16, dadurch gekennzeichnet, daß man ein oder mehrere Verbindungen einsetzt, die als Kolloid-Stabilisator und zugleich als Reduktionsmittel wirken.

18. Verfahren nach Anspruch 17, dadurch gekennzeichnet, daß man als Reduktionsmittel und Kolloid-Stabilisator K- Na-Citrat und/oder NH₄-Citrat einsetzt.

19. Verfahren nach einem oder mehreren der vorhergehenden Ansprüche,
dadurch gekennzeichnet, daß man den ersten Schritt und den zweiten Schritt nacheinander ausführt.

20. Verfahren nach Anspruch 19, dadurch gekennzeichnet, daß man den mit wenigstens einer Metallverbindung beaufschlagten porösen, vorzugsweise nanoporösen, Träger vor der Reduktion einem Trocknungsschritt unterwirft.

21. Verfahren nach einem oder mehreren der vorhergehenden Ansprüche 1 bis 18, dadurch gekennzeichnet, daß man den ersten Schritt und den zweiten Schritt in einem Eintopfverfahren ohne Isolierung, Reinigung oder Trocknung des beaufschlagten porösen, vorzugsweise nanoporösen, Trägers durchführt.

22. Verfahren nach einem oder mehreren der vorhergehenden Ansprüche, dadurch gekennzeichnet, daß man die Beaufschlagung und/oder Reduktion so führt, daß die Metallverbindungen in den Poren des Trägers in einer schalenförmigen oberflächennahen Zone zu den entsprechenden Metallen bzw. Legierungen in Form von ggf. stabilisierten Nanopartikeln unter Erhalt eines Schalenkatalysators reduziert werden.

23. Verfahren nach Anspruch 22, dadurch gekennzeichnet, daß man eine Schalendicke im Bereich zwischen 5 um und 5000 um erhält.

24. Verfahren nach einem oder mehreren der vorhergehenden Ansprüche, dadurch gekennzeichnet, daß man Katalysatoren erhält, die in den Poren und/oder der Schale metallene Partikel und/oder Legierungs-Partikel mit einem mittleren Teilchendurchmesser im Bereich von 1 bis 100 nm aufweisen.

25. Verfahren nach einem oder mehreren der vorhergehenden Ansprüche,
dadurch gekennzeichnet, daß man nach, vor oder während der Beaufschlagung und/oder Reduktion ein oder mehrere Aktivatoren und/oder Promotoren aufbringt.

26. Verfahren nach einem oder mehreren der vorhergehenden Ansprüche, dadurch gekennzeichnet, daß man die in-situ Reduktion bei Temperaturen zwischen Raumtemperatur und 150°C durchführt.

27. Verwendung des nach einem oder mehreren der vorhergehenden Ansprüche 22 - 26 erhältlichen Katalysators zur Herstellung von Vinylacetat in der Gasphase aus Ethylen, Essigsäure und Sauerstoff oder Sauerstoff enthaltenden Gasen.

## Claims

1. A process for producing a catalyst comprising one or more metals selected from the group of metals encompassing transition groups Ib and VIIIb of the Periodic Table of the Elements, with the exception of ruthenium, on porous support particles, which comprises, in a first step, applying one or more precursor(s) selected from the group of compounds consisting of the compounds of metals of transition groups Ib and VIIIb of the Periodic Table to a porous support;
and, in a second step, treating the porous support to which at least one precursor has been applied with at least one reducing agent to form a sol inside the pores of the support and to simultaneously fix said metal compound(s) on the support.

2. The process as claimed in claim 1, wherein an inert, porous, preferably nanoporous, support comprising silicon dioxide, aluminum oxide, titanium dioxide, zirconium dioxide, oxide mixtures of the compounds mentioned, mixed oxides of the compounds mentioned and/or aluminum silicates in the form of powders, sheets, strips, membranes, rods, plates, tablets, wagon wheels, monoliths, spheres, chips, rings, solid extrudates, hollow extrudates or stars is used.

3. The process as claimed in claim 1 or 2, wherein the metal compound(s) is/are applied to the porous support by steeping, spraying, dipping, impregnation, spray drying, hi-coating or fluidized-bed coating, preferably by impregnation.

4. The process as claimed in one or more of the preceding claims, wherein one or more compound(s) of metals selected from the group consisting of copper, silver, gold. iron, cobalt, nickel, rhodium, osmium, iridium, palladium and platinum is/are applied to the support.

5. The process as claimed in one or more of the preceding claims, wherein one or more palladium compound(s) alone or one or more palladium compound(s) together with one or more compound(s) of metals selected from the group consisting of copper, silver, gold. iron, cobalt, nickel, rhodium, osmium, iridium and platinum is/are applied to the porous support.

6. The process as claimed in one or more of the preceding claims, wherein one or more palladium compound(s) together with one or more compound(s) of gold are applied to the porous support.

7. The process as claimed in one or more of the preceding claims, wherein one or more reducing agents selected from the group consisting of citrates such as potassium citrate, sodium citrate, ammonium citrate; hydrazine, hydroxylamine, sodium hypophosphite, alkali metal borohydrides such as sodium borohydride, potassium borohydride; gaseous reducing agents such as hydrogen, carbon monoxide; formaldehyde, formates, acetates, oxalates, sulfanilates such as sodium hydroxymethanesulfinate; and monohydric or dihydric alcohols such as ethanol, ehtylene glycol; are used.

8. The process as claimed in one or more of the preceding claims, wherein the reducing agent used is potassium citrate, sodium citrate and/or ammonium citrate.

9. The process as claimed in one or more of the preceding claims, wherein a solution of the metal compound(s) is applied to the porous, preferably nanoporous, support.

10. The process as claimed in claim 9, wherein an aqueous solution, a solution in an organic solvent or a mixture thereof is applied to the support.

11. The process as claimed in claim 10, wherein water is used as solvent.

12. The process as claimed in claim 10, wherein methanol, ethanol, ethylene glycol, N-methylpyrrolidone, dimethylformamide, dimethylacetamide and/or tetrahydrofuran or a mixture of one or more of these substances with water is used as organic solvent.

13. The process as claimed in one or more of the preceding claims 9 to 12, wherein soluble palladium compounds, in particular water-soluble salts, selected from a group of Pd precursors consisting of palladium(II) acetate, palladium(II) chloride, palladium(II) nitrate and sodium tetrachloropalladate(II) [Na₂PdCl₄] are used.

14. The process as claimed in one or more of the preceding claims 9 to 13, wherein soluble metal compounds, in particular water-soluble salts, selected from a group of metal precursors consisting of tetrachloroauric(III) acid, gold(III) acetate [Au(OAc)₃], potassium aurate [KAuO₂], hexachloroplatinic(IV) acid hydrate, hexachloroiridic(IV) acid hydrate and rhodium(III) chloride hydrate are used.

15. The process as claimed in one or more of the preceding claims, wherein the application of the precursor(s) to the porous, preferably nanoporous, support and/or the reduction of the support to which the precursors) has/have been applied are/is carried out in the presence of a colloid stabilizer or a plurality of colloid stabilizers.

16. The process as claimed in claim 15, wherein one or more compound(s) selected from the group consisting of betaines, PVP, citrates, oxalates, formates, acetates, sulfanilates, PVA and PAA is/are added as colloid stabilizer.

17. The process as claimed in claim 15 or 16, wherein use is made of one or more compounds which simultaneously act as colloid stabilizer and as reducing agent.

18. The process as claimed in claim 17, wherein potassium citrate, sodium citrate and/or ammonium citrate is/are used as reducing agent and colloid stabilizer.

19. The process as claimed in one or more of the preceding claims, wherein the first and second steps are carried out successively.

20. The process as claimed in claim 19, wherein the porous, preferably nanoporous, support to which at least one metal compound has been applied is subjected to a drying step prior to the reduction.

21. The process as claimed in one or more of the preceding claims 1 to 18, wherein the first and second steps are carried out in a single-vessel process without isolation, purification or drying of the porous, preferably nanoporous, support to which the precursors) has/have been applied.

22. The process as claimed in one or more of the preceding claims, wherein the application of the precursor(s) and/or the reduction are/is carried out in such a way that the metal compounds are reduced in the pores of the support in a shell-like zone close to the surface to give the corresponding metals or alloys in the form of stabilized or unstabilized nanosize particles to produce a coated catalyst.

23. The process as claimed in claim 22, wherein a shell thickness in the range from 5 µm to 5000 µm is obtained.

24. The process as claimed in one or more of the preceding claims, wherein catalysts which have metal particles and/or alloy particles which have a mean particle diameter in the range from 1 to 100 nm in the pores and/or the shell are obtained.

25. The process as claimed in one or more of the preceding claims, wherein one or more activators and/or promoters is/are applied after, before or during the application of the precursor(s) and/or the reduction.

26. The process as claimed in one or more of the preceding claims, wherein the in-situ reduction is carried out at temperatures from room temperature to 150°C.

27. The use of the catalyst obtainable as claimed in one or more of the preceding claims 22-26 for preparing vinyl acetate in the gas phase from ethylene, acetic acid and oxygen or oxygen-containing gases.

## Revendications

1. Procédé de préparation d'un catalyseur contenant, sur des particules de support poreuses, un ou plusieurs métaux du groupe de métaux constitué par les groupes secondaires Ib et VIIIb du système périodique des éléments, caractérisé en ce que, dans une première étape, on applique sur un support poreux au moins une solution d'au moins un composé d'un métal du groupe des métaux des groupes secondaires Ib et VIIIb du système périodique, à l'exception du ruthénium, en tant qu'un ou plusieurs précurseurs du groupe de ces métaux, et en ce que, dans une deuxième étape, on traite le support poreux chargé d'au moins un précurseur avec au moins un agent réducteur de façon à produire un sol dans les pores du support par réduction du composé métallique et à le fixer sur le support dans cette étape.

2. Procédé selon la revendication 1, caractérisé en ce que l'on utilise un support inerte poreux, de préférence nanoporeux, constitué de dioxyde de silicium, d'oxyde d'aluminium, de dioxyde de titane, de dioxyde de zirconium, de mélanges d'oxydes des composés précités, d'oxydes mixtes des composés précités et/ou de silicates d'aluminium, sous forme de poudres, de feuilles, de bandes, de membranes, de cordons, de plaques, de pastilles, de roues de voiture, de monolithes, de billes, de copeaux, d'anneaux, d'extrudats pleins, d'extrudats creux ou d'étoiles.

3. Procédé selon la revendication 1 ou 2, caractérisé en ce que l'on applique le ou les composés métalliques sur le support poreux par imbibition, pulvérisation, immersion, imprégnation, séchage par pulvérisation, enrobage de type *hicoating* ou enrobage en lit fluidisé, de préférence par imprégnation.

4. Procédé selon l'une ou plusieurs des revendications précédentes, caractérisé en ce que l'on applique sur le support un ou plusieurs composés de métaux du groupe constitué par le cuivre, l'argent, l'or, le fer, le cobalt, le nickel, le rhodium, l'osmium, l'iridium, le palladium et le platine.

5. Procédé selon l'une ou plusieurs des revendications précédentes, caractérisé en ce que l'on applique sur le support poreux un ou plusieurs composés du palladium seuls ou un ou plusieurs composés du palladium avec un ou plusieurs composés de métaux du groupe constitué par le cuivre, l'argent, l'or, le fer, le cobalt, le nickel, le rhodium, l'osmium, l'iridium et le platine.

6. Procédé selon l'une quelconque des revendications précédentes, caractérisé en ce que l'on applique sur le support poreux un ou plusieurs composés de palladium avec un ou plusieurs composés d'or.

7. Procédé selon l'une ou plusieurs des revendications précédentes, caractérisé en ce que l'on utilise un ou plusieurs agents réducteurs du groupe constitué par des citrates comme le citrate de potassium, le citrate de sodium, le citrate d'ammonium; l'hydrazine, l'hydroxylamine, l'hypophosphite de sodium, des borohydrures de métaux alcalins comme le borohydrure de sodium, le borohydrure de potassium; des agents réducteurs gazeux comme l'hydrogène, le monoxyde de carbone; le formaldéhyde, des formiates, des acétates, des oxalates, des sulfanilates appropriés comme le sel de sodium de l'acide hydroxyméthanesulfinique; et des mono- ou dialcools comme l'éthanol, l'éthylèneglycol.

8. Procédé selon l'une ou plusieurs des revendications précédentes, caractérisé en ce que l'on utilise comme agent réducteur du citrate de potassium, du citrate de sodium et/ou du citrate d'ammonium.

9. Procédé selon l'une ou plusieurs des revendications précédentes, caractérisé en ce que l'on effectue l'application sur le support poreux, de préférence nanoporeux, en utilisant une solution du ou des composés métalliques.

10. Procédé selon la revendication 9, caractérisé en ce que l'on utilise pour l'application une solution aqueuse, une solution dans un solvant organique ou un de leurs mélanges.

11. Procédé selon la revendication 10, caractérisé en ce que l'on utilise de l'eau comme solvant.

12. Procédé selon la revendication 10, caractérisé en ce que l'on utilise comme solvant organique du méthanol, de l'éthanol, de l'éthylèneglycol, de la N-méthylpyrrolidone, du diméthylformamide, du diméthylacétamide et/ou du tétrahydrofurane ou un mélange d'un ou plusieurs de ces solvants avec de l'eau.

13. Procédé selon l'une ou plusieurs des revendications 9 à 12 précédentes, caractérisé en ce que l'on utilise des composés solubles du palladium, en particulier des sels solubles dans l'eau appartenant au groupe de précurseurs de palladium constitué par l'acétate de palladium-II, le chlorure de palladium-II, le nitrate de palladium-II et le palladium-II tétrachlorure de sodium [Na₂PdCl₄].

14. Procédé selon l'une ou plusieurs des revendications 9 à 13 précédentes, caractérisé en ce que l'on utilise des composés solubles de métaux, en particulier des sels solubles dans l'eau, appartenant au groupe de précurseurs de métaux constitué par l'acide aurichlorhydrique, l'acétate d'or-III [Au(OAc)₃], l'aurate de potassium [KAuO₂], l'acide platine-IV hexachlorhydrique hydraté, l'acide iridium-III hexachlorhydrique et/ou le chlorure de rhodium-III hydraté.

15. Procédé selon l'une ou plusieurs des revendications précédentes, caractérisé en ce que l'on effectue l'application sur le support poreux, de préférence nanoporeux, et/ou la réduction du support chargé en présence d'un stabilisant de colloïdes ou de plusieurs stabilisants de colloïdes.

16. Procédé selon la revendication 15, caractérisé en ce que l'on ajoute comme stabilisant de colloïdes un ou plusieurs composés du groupe constitué par des bétaïnes, des PVP, des citrates, des oxalates, des formiates, des acétates, des sulfanilates, des PVA et des PAA.

17. Procédé selon la revendication 15 ou 16, caractérisé en ce que l'on utilise un ou plusieurs composés qui agissent en même temps comme stabilisants de colloïdes et comme agents réducteurs.

18. Procédé selon la revendication 17, caractérisé en ce que l'on utilise comme agent réducteur et stabilisant de colloïdes du citrate de K - Na et/ou du citrate de NH₄.

19. Procédé selon l'une ou plusieurs des revendications précédentes, caractérisé en ce que l'on effectue successivement la première et la deuxième étape.

20. Procédé selon la revendication 19, caractérisé en ce que l'on soumet le support poreux, de préférence nanoporeux, chargé d'au moins un composé métallique à une étape de séchage avant la réduction.

21. Procédé selon l'une ou plusieurs des revendications 1 à 18 précédentes, caractérisé en ce que l'on effectue la première étape et la deuxième étape dans un procédé en un seul récipient sans isolement, purification ni séchage du support poreux, de préférence nanoporeux, chargé.

22. Procédé selon l'une ou plusieurs des revendications précédentes, caractérisé en ce que l'on effectue l'application et/ou la réduction de façon à réduire les composés métalliques dans les pores du support, dans une zone proche de la surface formant une coquille, en les métaux ou alliages correspondants sous forme de nanoparticules éventuellement stabilisées, en obtenant un catalyseur à coquille.

23. Procédé selon la revendication 22, caractérisé en ce que l'on obtient une épaisseur de coquille comprise entre 5 µm et 5 000 µm.

24. Procédé selon l'une ou plusieurs des revendications précédentes, caractérisé en ce que l'on obtient des catalyseurs qui présentent dans les pores et/ou la coquille des particules de métaux et/ou des particules d'alliage ayant un diamètre de particule moyen compris entre 1 et 100 nm.

25. Procédé selon l'une ou plusieurs des revendications précédentes, caractérisé en ce que, après, avant ou pendant l'application et/ou la réduction, on applique un ou plusieurs activateurs et/ou un ou plusieurs promoteurs.

26. Procédé selon l'une ou plusieurs des revendications précédentes, caractérisé en ce que l'on effectue la réduction *in situ* à des températures comprises entre la température ambiante et 150°C.

27. Utilisation du catalyseur pouvant être obtenu selon l'une ou plusieurs des revendications 22 à 26 précédentes pour la préparation d'acétate de vinyle en phase gazeuse à partir d'éthylène, d'acide acétique et d'oxygène ou de gaz contenant de l'oxygène.
